# DEMANDE DE BREVET EUROPEEN

(11) **EP 4 407 626 A1**
(43) Date de publication de la demande: **31.07.2024**
(21) Numéro de dépôt: 23154031.1
(22) Date de dépôt: 30.01.2023
(51) Int. Cl.: G16H 10/20, G16H 50/30

(54) **PROCÉDÉ DE GÉNÉRATION DE RECOMMANDATIONS DIAGNOSTIQUES ET/OU THÉRAPEUTIQUES EN FERTILITÉ**

(71) Demandeur: SOFIA, 33130 Begles (FR)
(72) Inventeur: GRIMAL, Anaïs, 24440 MONTFERRAND DU PERIGORD (FR); BOUDIR, Atlal, 33000 BORDEAUX (FR)
(74) Mandataire: Aquinov

(57) **Abrégé**

L'invention concerne un procédé de génération de recommandations pour un parcours de procréation médicalement assisté d'un patient mis en oeuvre par un système informatique comportant un serveur de traitement et deux terminaux informatiques ; le procédé comportant les étapes suivantes : étape de fourniture d'un ensemble de données cliniques et de données contextuelles et/ou psycho-sociales du patient ; étape d'identification, par ledit serveur de traitement, de facteurs de risque d'infertilité à partir des données cliniques, contextuelles et/ou psycho-sociales fournies par le patient ; étape de génération, par ledit serveur de traitement, de recommandations de santé à partir des facteurs de risque d'infertilité identifiés et des données cliniques, contextuelles et/ou psycho-sociales fournies ; étape de transmission desdites données cliniques, contextuelles et/ou psycho-sociales, des facteurs de risque d'infertilité identifiés et des recommandations générées par le serveur de traitement audit deuxième terminal informatique.

## Description

### Domaine technique.

L'invention se rapporte au domaine technique des outils d'aide à la décision en contexte médical et à celui de procédés d'amélioration de la mémorisation et l'apprentissage d'informations par des patients.

L'invention a pour objet un procédé de génération automatique de recommandations d'un parcours de procréation médicalement mis en oeuvre par un système informatique et capable d'augmenter le score de littératie en santé chez un patient suivant, s'apprêtant à suivre ou ayant déjà suivi un ou plusieurs parcours de procréation médicalement assisté.

### Etat de la technique.

Il est bien établi que la littératie en matière de santé, notamment en matière procréation médicalement assistée (PMA), est un facteur clé dans la réussite de la PMA. Les patients qui ont un niveau de littératie en santé élevé sont mieux informés sur les différents parcours de PMA disponibles, les mécanismes biologiques impliqués dans le processus de conception et les risques éventuels associés à la PMA. Cette connaissance permet aux patients de prendre des décisions éclairées et de participer activement à leur parcours de PMA, ce qui augmente leur chance de réussite.

Cependant, augmenter le niveau de littératie en matière de PMA peut être un défi, car il existe une grande quantité d'informations disponibles sur le sujet. Les patients peuvent avoir du mal à trouver des informations pertinentes pour leur cas particulier et/ou rencontrer des difficultés pour mémoriser et intégrer toutes ces informations. Les informations disponibles sur internet peuvent également être dépassées ou même erronées, rendant la tâche encore plus difficile.

Pour surmonter ces défis, il est important que les patients aient accès à des sources d'informations fiables et actualisées, telles que les sites web des sociétés de procréation médicalement assistée, les associations de patients ou encore les organismes officiels de santé. Les professionnels de la santé peuvent également jouer un rôle clé en fournissant des informations précises et en répondant aux questions des patients. Il est également important de donner aux patients le temps et les outils nécessaires pour mémoriser et intégrer les informations ; classiquement, les patients ont accès à des feuilles d'informations imprimées ou des séances de suivi pour discuter des informations apprises. Néanmoins, face à une quantité importante d'information reçue en un temps limité, les patients n'arrivent pas à discerner facilement les informations essentielles et/ou les informations qui les concernent en particulier ni à les intégrer ; par ailleurs, le temps passé en accompagnement d'un professionnel de santé est souvent limité, de sorte que les patients n'ont pas nécessairement le temps de poser les questions qui pourraient surgir et/ou peuvent ne pas oser demander au professionnel de santé de réexpliquer un sujet déjà traité.

Par ailleurs, les protocoles et les technologies liés à la PMA évoluent constamment, ce qui signifie que les professionnels de la santé doivent être en mesure de rester à jour avec les dernières avancées pour offrir les meilleurs soins à leurs patients. Les professionnels de la santé peuvent également avoir besoin d'aide pour naviguer les complexités des différents parcours de PMA disponibles et pour prendre des décisions éclairées en tenant compte des risques et des bénéfices potentiels pour chaque patient.

De plus, les professionnels de la santé peuvent également avoir besoin d'un outil pour les aider à prendre des décisions plus rapidement et de manière plus efficace en gardant en tête les détails médicaux importants de chaque patient, lesquels peuvent être facilement oubliés. Les outils informatiques, comme les logiciels de gestion de dossiers médicaux, peuvent être très utiles pour gérer les informations médicales de chaque patient et pour prendre des décisions de manière plus efficace.

Dès lors, les professionnels de la santé ont également besoin d'une aide pour maintenir un niveau de littératie en santé élevé en matière de PMA, en suivant les nouveaux protocoles et les technologies récentes, en prenant des décisions éclairées en tenant compte des risques et des bénéfices potentiels pour chaque patient, et en utilisant des outils informatiques pour faciliter la gestion des dossiers médicaux pour éviter les oublis.

Ainsi, il existe un réel besoin d'augmenter le niveau de littératie en matière de PMA de sorte à augmenter le taux de réussite de la PMA tout en permettant au professionnel de santé en charge des patients d'être à jour sur les derniers protocoles existants en matière de PMA et de faciliter la prise en charge globale des patients au travers de la prise en compte de l'ensemble de leurs particularités. Cet objectif est un réel défi en raison de la grande quantité d'informations disponibles et de la difficulté à trouver et mémoriser les informations pertinentes. Il est important que les patients aient accès à des sources d'informations fiables et actualisées et que les professionnels de la santé jouent un rôle actif dans la fourniture d'informations précises et en répondant aux questions des patients.

L'invention se place donc dans ce contexte et cherche à résoudre l'ensemble des inconvénients précités. Ainsi, l'invention cherche à proposer un procédé de recommandations pour un parcours de procréation médicalement assisté d'un patient permettant d'augmenter un score de littératie en santé dudit patient et de générer des recommandations médicales pour le professionnel de santé en charge du suivi médical dudit patient.

### Présentation de l'invention.

L'invention a pour objet un procédé de recommandations pour un parcours de procréation médicalement assisté d'un patient, le procédé étant mis en oeuvre par un système informatique comportant : un serveur de traitement comportant une mémoire de stockage, un premier terminal informatique et un deuxième terminal informatique, les deux terminaux informatiques étant aptes à communiquer avec ledit serveur de traitement.

Le procédé de génération de recommandations pour un parcours de procréation médicalement assisté d'un patient comporte les étapes suivantes :
a. étape de fourniture d'un ensemble de données cliniques et de données contextuelles et/ou psycho-sociales du patient, au moins une partie desdites données cliniques et desdites données contextuelles et/ou psycho-sociales étant fournie au travers d'un questionnaire exécuté par le patient au moyen dudit premier terminal informatique ;
b. étape d'identification, par ledit serveur de traitement, d'un ou plusieurs facteurs de risque d'infertilité à partir des données cliniques et de données contextuelles et/ou psycho-sociales fournies par le patient, un ensemble de règles d'identification de risques d'infertilité étant stocké dans la mémoire de stockage, le ou lesdits facteurs de risque d'infertilité étant déterminés par le serveur de traitement par application de tout ou partie desdites règles d'identification sur au moins une partie des données cliniques et de données contextuelles et/ou psycho-sociales ;
c. étape de génération, par ledit serveur de traitement, d'un ensemble de recommandations de santé à partir des facteurs de risque d'infertilité identifiés et des données cliniques et des données contextuelles et/ou psycho-sociales fournies ;
d. étape de transmission desdites données cliniques et desdites données contextuelles et/ou psycho-sociales, du ou des facteurs de risque d'infertilité identifiés et des recommandations générées par le serveur de traitement audit deuxième terminal informatique.

L'invention propose ainsi de générer les recommandations de santé personnalisées, sur la base des données cliniques et de données contextuelles et/ou psycho-sociales propres au patient, en procédant à une application directe, systématique et automatique d'un ensemble de règles d'identification de risques d'infertilité. En procédant ainsi, il est possible, d'une part, de prendre en compte et de traiter rapidement un grand nombre de configurations possibles résultant de la diversité de pathologies et de données cliniques et de données contextuelles et/ou psycho-sociales nécessaires pour établir lesdites recommandations de santé ; permettant ainsi d'adapter les traitements du patient en fonction de son historique médical et des facteurs de risque spécifiques qui peuvent influencer leur capacité à procréer.

Dans la présente invention, on entend par « infobulle » la donnée d'une quantité d'information réduite et facile à comprendre, affichée dans une interface utilisateur conviviale et conçue pour présenter des informations de manière concise et visuellement attrayante de sorte à communiquer des informations complexes de manière simple, compréhensible, ergonomique et accessible.

Dans la présente invention, on entend par « facteur de risque d'infertilité » toute condition pathologique et/ou environnementale, notamment liée au mode de vie d'une personne, qui augmente le risque de ladite personne de ne pas pouvoir concevoir ou de pouvoir mener une grossesse à terme.

Sont des facteurs de risque d'infertilité, notamment, l'âge avancé, les maladies chroniques telles que le diabète et l'hypertension, l'obésité, l'exposition à des toxines environnementales, les troubles hormonaux, les infections génitales, les maladies génétiques et les habitudes de vie comme le tabagisme, la consommation excessive d'alcool et le manque d'exercice physique. Les facteurs de risque peuvent varier selon le sexe et peuvent être différents entre les hommes et les femmes.

Dans la présente invention, on entend par « recommandation de santé », toute recommandation à des fins de diagnostic et/ou à des fins thérapeutiques visant à diminuer, voire éliminer, toute ou partie d'un ou plusieurs facteurs de risque d'infertilité. Il pourra notamment s'agir de toute suggestion et/ou orientation donnée en ce qui concerne les meilleures pratiques en matière de santé pour préserver, voire améliorer, la fertilité d'un individu et éviter les facteurs de risque, notamment de risque d'infertilité, qui peuvent l'affecter négativement.

Dans la présente invention, on entend par « parcours de diagnostic » d'un patient, un processus structuré utilisé pour identifier une maladie ou un problème de santé, notamment problème de fertilité, sur la base de symptômes, antécédents médicaux, examens médicaux tels que des tests de laboratoire ou tout autre information médicale.

Des recommandations de santé peuvent être, notamment, des conseils sur l'alimentation, l'utilisation de médicaments, pratique d'une activité sportive ; les recommandations peuvent également porter sur des stratégies de prévention, des options de traitement ou des critères de surveillance médicale tels que des analyses médicales, ainsi que des conseils bibliographiques tels que des articles scientifiques ou encore des directives officielles de santé publique en matière de protocoles médicaux de procréation et de fertilité.

Si on le souhaite, le premier terminal informatique peut être un ordinateur, fixe ou portable, une tablette numérique, un téléphone mobile munie d'un écran, ou encore, un téléviseur, notamment un téléviseur muni d'un écran tactile.

Avantageusement, lors de l'étape de fourniture d'un ensemble de données cliniques, le patient procède au renseignement dudit ensemble de données cliniques et de données contextuelles et/ou psycho-sociales au moyen d'une interface graphique affichée dans le premier terminal informatique.

Si on le souhaite, l'ensemble de données cliniques fournies comporte des informations du patient, notamment des informations parmi l'âge, le sexe, allergies connues, pathologies connues, antécédents médicaux, groupe sanguin, résultats d'examens tels que des bilans hormonaux et des tests de qualité spermatique, traitement en cours, handicaps, protocoles de procréation médicalement assistée déjà suivis, historique de traitement contraceptif, nom et/ou type des traitements contraceptifs utilisés, la durée d'utilisation desdits traitements contraceptifs, date de début de l'utilisation de traitements contraceptifs, date d'arrêt de l'utilisation de traitements contraceptifs, dates et nombre d'anciennes grossesses et le nombre de mois de conception associés auxdites anciennes grossesses, présence et type de complications lors d'une grossesse passée, nombre de partenaires sexuels, utilisation de drogues, consommation d'alcool, type d'activités sportives, maladies génétiques, historique de pathologies familiales, résultats de biochimie sanguine, résultats d'anciens spermogrammes, informations des difficultés d'érection, informations sur des pathologies testiculaires ou assimilées, nombre d'enfants, nombre d'accouchements prématurés, régularité du cycle menstruel, longueur du cycle menstruel, nombre de rapports sexuels hebdomadaires, historique de vaccination, historique sur des maladies sexuellement transmissibles.

On entend par « données contextuelles et/ou psycho-sociales » des données de type : état psychologique, contexte culturel, niveau d'éducation, niveau de revenu et statut économique, environnement, profession, structure familiale.

Avantageusement, au moins une partie desdites données cliniques et desdites données contextuelles et/ou psycho-sociales est renseignée au travers d'un questionnaire. Dans un exemple de réalisation, le questionnaire médical pourra être un questionnaire séquentiel dynamique. En d'autres termes, les questions du questionnaire pourront être exécutées séquentiellement, les unes après les autres ou par groupes successifs, l'exécution d'une question ou d'un groupe de questions dépendant des réponses fournies à tout ou partie des questions ou groupes de questions précédents.

La fourniture des réponses aux questions pourra être réalisée manuellement par le patient, notamment via un clavier du premier terminal informatique. En variante, les réponses pourront être dictées au premier terminal informatique, via un microphone, le premier terminal informatique étant muni d'une application logicielle de dictée et de reconnaissance vocale.

Dans un exemple de réalisation de l'invention, alternatif ou cumulatif, le premier terminal informatique est apte à afficher une première interface graphique, notamment destinée au patient.

Avantageusement, ladite première interface graphique permet d'afficher et de remplir le questionnaire dynamique, ainsi que d'exécuter ledit questionnaire dynamique sur le premier terminal informatique, de sorte que chaque question ou groupe de questions dudit questionnaire dynamique soit affiché sur tout ou partie de ladite première interface graphique.

Dans une mode de réalisation de l'invention, alternatif ou cumulatif, le questionnaire dynamique est déterminé au moyen d'algorithmes de logique conditionnelle permettant notamment de déterminer les questions devant être affichées en fonction des données préalablement inscrites. En procédant ainsi, il est possible de maximiser la pertinence des questions posées en les adaptant aux caractéristiques individuelles du patient et en évitant des questions inutiles ou répétitives ; en s'assurant que les questions sont appropriées pour le patient, il est possible d'améliorer la qualité des données recueillies tout en minimisant les éventuelles erreurs de saisie et/ou de compréhension.

Avantageusement, la première interface graphique comporte un accès à une messagerie sécurisée pour communiquer avec le médecin. Ladite première interface graphique peut présenter une variété d'options pour le patient, notamment, permettant de communiquer avec les professionnels de santé en charge du patient en temps réel ou en différé. En procédant ainsi, le patient a la possibilité de communiquer avec les professionnels de santé à distance, évitant par ce biais les déplacements et permettant un accès plus facile pour les patients vivant dans des régions éloignées ou ayant des besoins spécifiques.

Si on le souhaite, la première interface graphique comprend également des fonctionnalités pour la prise de rendez-vous, la gestion des médicaments et autres traitements thérapeutiques la gestion des résultats d'examens. Ladite première interface peut également comprendre des fonctionnalités d'alertes, notamment en cas d'un rendez-vous médical dont l'échéance est proche, de traitement arrivant à échéance ou de tests médicaux à réaliser.

Avantageusement, la première interface graphique prévoit la possibilité de téléverser et/ou télécharger des images, des fichiers audio et/ou des fichiers vidéo, notamment pour partager le contenu aux professionnels de santé.

Avantageusement, le système informatique comporte des méthodes de sécurisation et notamment de protection de la confidentialité des données, par exemple mis en oeuvre, notamment, au travers des méthodes de confidentialité différentielle tels que le chiffrement homomorphique.

Dans un exemple de réalisation de l'invention, alternatif ou cumulatif, la première interface graphique comporte une carte interactive, par exemple, sous forme de frise chronologique ou de chemin, permettant de visualiser les différentes étapes du parcours déjà suivies, telles que les dates de réalisation d'analyses biologiques, les dates de début et de fin de menstruation, les dates d'inséminations, les dates de début et fin de traitements thérapeutiques et les dates de consultations médicales.

Avantageusement, la carte interactive permet également de visualiser les événements médicaux prévus dans le futur, tels que les prochaines consultations avec un professionnel de santé ou les dates des prochains traitements devant être suivis. Si on le souhaite, les informations du patient pourront être représentées, notamment, au travers d'icônes, notamment des graphiques en forme de jauge ou de barre de progression, et/ou des codes de couleurs permettant d'identifier rapidement les différents types d'événements et une ou plusieurs des caractéristiques desdits évènements.

Dans un mode de réalisation de l'invention, la carte interactive permet de générer un ensemble de parcours envisageables, de sorte que chaque parcours envisageable soit constitué d'un ensemble d'étapes, notamment des analyses de laboratoire et/ou des diagnostics annexes, envisageables au regard des protocoles de fertilité existants.

Dans une variante de ce mode de réalisation, le questionnaire est exécuté sur le serveur de traitement depuis le premier terminal informatique. Cela permet de réduire les risques d'erreurs de saisie ou de transmission des données et de faciliter la collecte des informations auprès des patients. Cette approche permet également de limiter les déplacements du patient pour remplir le questionnaire, en facilitant la participation du patient à distance.

Dans un mode de réalisation de l'invention, l'étape de fourniture d'un ensemble de données cliniques et de données contextuelles et/ou psycho-sociales comprend une sous étape de récolte de données, dites données autogénérées, relatives à l'utilisation faite par le patient du premier terminal informatique lors de l'exécution du questionnaire dynamique. Avantageusement, les données récoltées pourront notamment être des mesures du temps passé sur une question donnée, l'enregistrement de l'historique de navigation dans le questionnaire et, notamment, à partir du déplacement à travers les différentes questions du questionnaire, l'historique de navigation sur un moteur de recherche, horaire de connexion et de remplissage du questionnaire, nombre de connexions. Ces données autogénérées fournissent des informations de la compréhension et l'interaction du patient avec le questionnaire, ce qui permet d'identifier des points de confusion ou des lacunes dans les questions. En outre, ces données aident à évaluer la pertinence et la qualité des questions.

Dans un exemple de réalisation de l'invention, la fourniture de tout ou partie des données cliniques et de données contextuelles et/ou psycho-sociales du patient déclenche le calcul d'un score de littératie en santé, ledit score de littératie en santé étant associé au patient est stockée sur une mémoire dudit premier terminal informatique ou, en variante, sur la mémoire de stockage du serveur de traitement. En procédant ainsi, il est possible d'identifier les patients qui ont besoin d'une assistance supplémentaire pour comprendre les informations médicales et les recommandations de santé, et de les orienter vers des ressources appropriées.

Avantageusement, la valeur du score de littératie d'un patient est recalculée et mise à jour dans la mémoire chargée de sauvegarder sa valeur chaque fois que le patient interagit avec la première interface graphique exécutée dans le premier terminal informatique.

Dans un mode de réalisation de l'invention, la première interface graphique est apte à afficher un ou plusieurs éléments graphiques, notamment des éléments graphiques comportant des synthèses d'informations de santé, visant à améliorer la compréhension du patient en ce qui concerne son parcours de fertilité et, notamment, à améliorer son score de littératie en santé.

Avantageusement, les éléments graphiques affichés sur la première interface sont établis sur la base du score de littératie en santé associé au patient et à l'ensemble de données cliniques, personnelles et/ou contextuelles.

Dans un mode de réalisation de l'invention, le serveur de traitement est apte à envoyer un signal d'affichage au premier terminal informatique sur la base de la valeur du score de littératie en santé.

Si on le souhaite, lorsque le résultat du score de littératie en santé dépasse un seuil numérique donné, la première interface graphique est apte à afficher des éléments graphiques indiquant au patient sa valeur de littératie en santé associée, ou une fonction de ladite valeur de littératie en santé. Il pourra s'agir notamment d'une attribution de points de récompense, d'un nombre d'étoiles, ou de tout autre élément assimilé. En procédant ainsi, le patient est motivé à poursuivre le développement de son score de littératie en santé, notamment en vue d'augmenter les chances de réussite d'un parcours de procréation médicalement assisté.

Dans un mode de réalisation de l'invention, alternatif ou cumulatif, le serveur de traitement détermine le score de littératie en santé du patient de sorte que, en fonction dudit score et des données cliniques et des données contextuelles et/ou psycho-sociales fournies, le serveur est apte à générer une instruction d'affichage d'une infobulle. Ladite infobulle contient des informations sur les recommandations générées pour le patient en fonction des données cliniques et des données contextuelles et/ou psycho-sociales et, le cas échéant, des anciennes valeurs du score de littératie en santé.

Dans un mode de réalisation de l'invention, alternatif ou cumulatif, le procédé comporte une étape de réception par le serveur de traitement, préalablement à l'étape d'identification de risques de fertilité, d'un deuxième ensemble de données cliniques et de données contextuelles et/ou psycho-sociales du patient, notamment des données cliniques et de données contextuelles et/ou psycho-sociales d'un profil national de santé associé au patient.

Si on le souhaite, la transmission dudit deuxième ensemble de données cliniques et de données contextuelles et/ou psycho-sociales du patient peut être effectuée par un serveur de traitement distant.

Dans un mode de réalisation de l'invention, alternatif ou cumulatif, le procédé comprend une étape de transmission des données fournies via le questionnaire au serveur de traitement. Cette étape permet de centraliser les informations collectées auprès du patient et de les traiter de manière efficace en vue de générer les recommandations de santé.

Dans un mode de réalisation de l'invention, le système informatique comporte un ensemble de règles d'identification de risques d'infertilité. Ledit ensemble de règles d'identification étant stocké dans la mémoire de stockage du serveur de traitement ou, en variante, sur une mémoire de stockage distante accessible par ledit serveur de traitement.

Avantageusement, chaque règle dudit ensemble de règles d'identification de risques de fertilité peut être appliquée sur l'ensemble des données fournies par le patient au moyen du questionnaire dynamique de sorte à produire un ensemble, éventuellement vide, de risques de fertilité.

Avantageusement, le système informatique comporte une bibliothèque de facteurs de risque d'infertilité prédéterminés, ladite bibliothèque étant stockée dans la mémoire de stockage du serveur de traitement. La bibliothèque de facteurs de risque d'infertilité prédéterminés étant agencée de sorte que toute règle d'identification de risques de fertilité associe un ensemble de types de données cliniques et de données contextuelles et/ou psycho-sociales à un ou plusieurs facteurs de risque d'infertilité prédéterminés et à un parcours de diagnostic d'un patient.

Dans un mode de réalisation de l'invention, l'étape d'identification d'un ou plusieurs facteurs de risque d'infertilité comporte une sous-étape de sélection d'une ou plusieurs règles d'identification parmi l'ensemble de règles d'identification.

Dans un mode de réalisation alternatif de l'invention la sous-étape de sélection d'une ou plusieurs règles d'identification parmi ledit ensemble de règles d'identification à partir de tout ou partie des données cliniques et de données contextuelles et/ou psycho-sociales fournies par le patient est exécutée par un algorithme de type système expert, notamment ayant recours à un moteur d'inférence. Le moteur d'inférence pouvant être un moteur d'inférence à chaînage avant, à chaînage arrière ou à chaînage mixte.

En variante, la sous-étape de sélection d'une ou plusieurs règles d'identification parmi ledit ensemble de règles d'identification à partir de tout ou partie des données cliniques et de données contextuelles et/ou psycho-sociales fournies par le patient est exécutée par un algorithme de type apprentissage machine de type traitement automatique du langage, notamment apprentissage profond employant des modèles génératifs basés sur des réseaux de neurones.

En variante, la sous-étape de sélection d'une ou plusieurs règles d'identification parmi ledit ensemble de règles d'identification à partir de tout ou partie des données cliniques et de données contextuelles et/ou psycho-sociales fournies par le patient est exécutée par un algorithme de type règles booléennes.

Dans un mode de réalisation cumulatif de l'invention, l'étape d'identification d'un ou plusieurs facteurs de risque d'infertilité comporte une sous-étape, dite sous-étape de filtrage, consécutive de la sous-étape de sélection, effectuant un filtrage, à partir des règles d'identification sélectionnées, de facteurs de risque d'infertilité prédéterminés de la bibliothèque de facteurs de risque d'infertilité prédéterminés pour identifier le ou lesdits facteurs de risque associés au patient.

Dans un mode de réalisation de l'invention, le système informatique comporte une base de recommandations de santé, notamment stockée dans la mémoire de stockage du serveur de traitement ou, en variante, sur une mémoire de stockage distante accessible par ledit serveur de traitement. Avantageusement, le recours à une base de recommandations de santé permet d'avoir un accès centralisé et rapide à un grand nombre d'informations médicales ; de plus, les recommandations de santé peuvent être mises à jour facilement, ce qui garantit que les informations sont toujours à jour et précises.

Si on le souhaite, la base de recommandations de santé peut comporter, notamment, des références à des articles scientifiques et/ou tout ou partie desdits articles scientifiques, des références à des directives de santé publique et/ou tout ou partie desdites directives de santé publique.

Comme spécifié précédemment, le procédé comporte une étape de génération par le serveur de traitement d'un ensemble de recommandations de santé à partir des facteurs de risque d'infertilité identifiés et de l'ensemble données cliniques et de données contextuelles et/ou psycho-sociales fourni.

Avantageusement, l'étape de génération de recommandations de santé comprend une première sous-étape d'identification d'étiquettes à partir des facteurs de risque d'infertilité identifiés. Les étiquettes identifiées permettent de synthétiser et d'encoder un contenu sémantique clé, notamment des facteurs de risque d'infertilité, et de s'y référer si besoin.

Avantageusement, l'étape de génération de recommandations de santé comprend une deuxième sous-étape de sélection, consécutive à la première sous-étape d'identification d'étiquettes, d'une ou plusieurs recommandations de santé parmi la base de recommandations de santé prédéterminées.

Dans un mode de réalisation cumulatif de l'invention, la sous-étape de sélection d'une ou plusieurs recommandations de santé est réalisée au moyen d'une méthode de maximisation d'un degré de correspondance sémantique. En procédant ainsi, il est possible de trouver les correspondances les plus pertinentes entre les données médicales du patient et les recommandations de santé. De plus, grâce à ce type de méthodes algorithmiques il est possible d'accélérer la recherche des recommandations de santé les plus pertinentes pour le patient et de maximiser la fiabilité des recommandations de santé obtenues.

Si on le souhaite, la méthode de maximisation d'un degré de correspondance sémantique pourra être une méthode de filtrage basée sur le contenu, une méthode de filtrage collaboratif, une méthode basée sur les graphes ou encore une méthode hybride composant plusieurs des méthodes précédentes.

Comme indiqué précédemment, le procédé comporte une étape de transmission, par le serveur de traitement, des recommandations de santé générées, de l'ensemble de données cliniques et de données contextuelles et/ou psycho-sociales fourni et de l'ensemble des facteurs de risques d'infertilité identifiés, vers le deuxième terminal informatique.

Dans un mode de réalisation de l'invention, le deuxième terminal informatique comporte une deuxième interface graphique, notamment à destination du médecin en charge du suivi du patient. Ladite deuxième interface graphique permettant notamment l'affichage d'un tableau de bord contenant les données cliniques et de données contextuelles et/ou psycho-sociales et/ou des risques de fertilité identifiés et/ou des recommandations de santé générées par le serveur de traitement.

Avantageusement, la deuxième interface graphique est agencée pour résumer graphiquement l'ensemble des données transmises par le serveur de traitement sur un ou plusieurs composants graphiques, dits résumés graphiques.

Le ou les résumés graphiques des données fournies pouvant comporter des graphes de valeurs temporelles, notamment une représentation temporelle de l'ensemble des parcours de diagnostics effectués par le patient. En procédant ainsi, la représentation temporelle des parcours de diagnostic permet de simplifier la compréhension du parcours de santé du patient, de le situer rapidement sur ledit parcours et de visualiser les différentes étapes consécutives, contribuant ainsi à améliorer globalement la rapidité d'évaluation médicale par le professionnel de santé.

Si on le souhaite, la deuxième interface graphique peut également comporter un composant graphique représentant les facteurs de risque d'infertilité identifiés par le serveur de traitement et un troisième composant graphique représentant les recommandations générées par le serveur de traitement.

En procédant ainsi, il est possible d'avoir une vue globale et claire des données relatives à un patient, notamment les parcours de diagnostic effectués, les facteurs de risque d'infertilité identifiés et les recommandations générées. Cela facilite la prise de décisions médicales par le professionnel de santé et permet de mieux cibler les facteurs de risque pertinents pour le patient afin d'optimiser les chances de réussite de la procréation médicalement assistée.

### Brève description des figures.

D'autres avantages et caractéristiques de la présente invention sont maintenant décrits à l'aide d'exemples uniquement illustratifs et nullement limitatifs de la portée de l'invention, et à partir des dessins annexés, dessins sur lesquels les différentes figures représentent :
[Fig. 1] représente, schématiquement et partiellement, un système informatique pour la mise en oeuvre d'un procédé selon un exemple de réalisation de l'invention.
[Fig. 2] représente, schématiquement et partiellement, un procédé de transmission d'un compte-rendu d'un examen médical selon un exemple de réalisation de l'invention, mis en oeuvre au moyen du système informatique de la [Fig. 1].
[Fig. 3] représente, schématiquement et partiellement, une interface graphique affichée sur le premier terminal numérique selon un mode de réalisation de l'invention.
[Fig. 4] représente, schématiquement et partiellement, une interface graphique affichée sur le deuxième terminal numérique selon un mode de réalisation de l'invention.

Dans la description qui suit, les éléments identiques, par structure ou par fonction, apparaissant sur différentes figures conservent, sauf précision contraire, les mêmes références.

Les procédés qui vont être décrits peuvent également être mises en oeuvre par des programmes logiciels exécutables par un système informatique. En outre, leur mise en oeuvre pourra indifféremment être réalisé par un traitement distribué et/ou un traitement parallèle, notamment pour traiter en parallèle plusieurs données.

Les figures décrites dans le présent document sont destinées à fournir une compréhension générale de l'invention sous divers modes de réalisation. Ces figures ne sont pas destinées à servir de description complète de tous les éléments et caractéristiques des appareils, processeurs et systèmes nécessaires à l'invention. De nombreux autres modes de réalisation de l'invention, ou des combinaisons de ceux-ci peuvent être apparents pour l'homme du métier à la lecture de cette description, en combinant les modes de réalisation divulgués. D'autres modes de réalisation peuvent être dérivés de la description, de sorte que des substitutions et des changements structurels et logiques peuvent être effectués sans s'écarter du cadre de la présente invention.

En outre, la description et les figures doivent être considérées comme illustratives plutôt que restrictives, et les revendications annexées sont destinées à couvrir toutes les modifications, améliorations et autres modes de réalisation de l'invention. Ainsi, la portée des revendications suivantes doit être déterminée par l'interprétation la plus large possible des revendications et de leurs équivalents et ne doit pas être restreinte ou limitée par la description qui précède.

### Description des modes de réalisation.

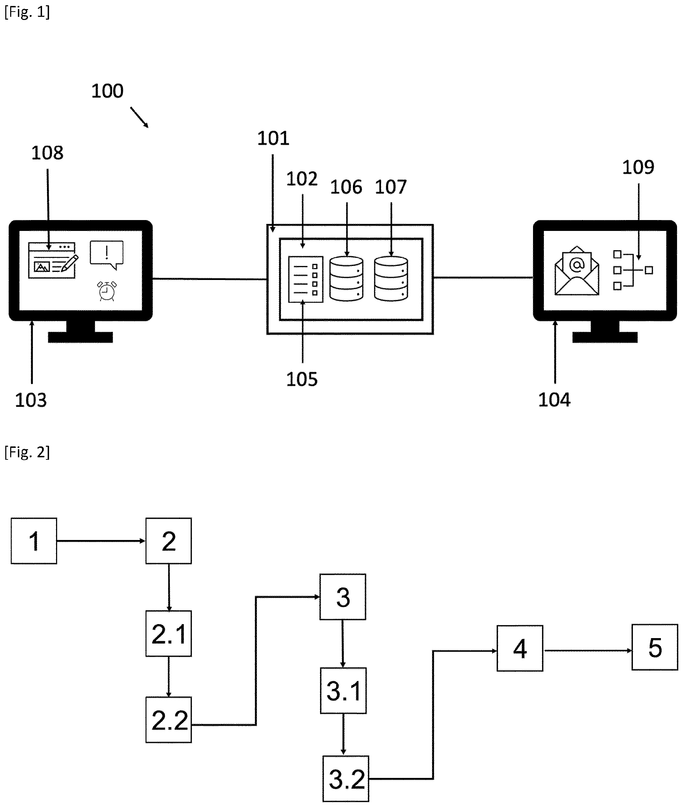

On a représenté en [Fig. 1] un système informatique 100 pour la mise en oeuvre du procédé de génération de recommandations pour un parcours de procréation médicalement assisté selon la [Fig. 2]. Le système informatique comporte un premier terminal informatique 103 et un deuxième terminal informatique 104 et un serveur de traitement 101 apte à communiquer avec ledit premierterminal informatique 103 et avec ledit deuxième terminal informatique 104.

Le serveur de traitement 101 comporte une mémoire de stockage 102 permettant de stocker une base de règles d'identification de facteurs de risques d'infertilité 105, une bibliothèque de facteurs de risque d'infertilité 106 et une base de recommandations de santé 107.

On a représenté en [Fig. 2] un schéma block d'un procédé de recommandations pour un parcours de procréation médicalement assisté d'un patient, selon un mode de réalisation de l'invention comportant une étape 1 de fourniture d'un ensemble de données cliniques et de données contextuelles et/ou psycho-sociales d'un patient, au moins une partie des données cliniques et de données contextuelles et/ou psycho-sociales étant fournie au travers d'un questionnaire séquentiel dynamique 108 exécuté par le patient au moyen du premier terminal informatique 103.

La fourniture des réponses aux questions du questionnaire séquentiel dynamique 108 est réalisée manuellement par le patient, via un clavier (non représenté) du premier terminal informatique 103.

Le questionnaire dynamique 108 est déterminé au moyen d'algorithmes de logique conditionnelle permettant notamment de déterminer les questions devant être affichées en fonction des données préalablement inscrites.

Lors de l'étape 1 de fourniture d'un ensemble de données cliniques, le patient procède au renseignement dudit ensemble de données cliniques et de données contextuelles et/ou psycho-sociales au moyen d'une interface graphique 200 affichée dans le premier terminal informatique 103.

L'ensemble de données cliniques fournies comporte des informations du patient, notamment des informations sur l'âge, le sexe, allergies connues, pathologies connues, antécédents médicaux, groupe sanguin, résultats d'examens tels que des bilans hormonaux et des tests de qualité spermatique, traitement en cours, handicaps, protocoles de procréation médicalement assistée déjà suivis, historique de traitement contraceptif, nom et/ou type des traitements contraceptifs utilisés, la durée d'utilisation desdits traitements contraceptifs, date de début de l'utilisation de traitements contraceptifs, date d'arrêt de l'utilisation de traitements contraceptifs, dates et nombre d'anciennes grossesses et le nombre de mois de conception associés auxdites anciennes grossesses, présence et type de complications lors d'une grossesse passée, nombre de partenaires sexuels, utilisation de drogues, consommation d'alcool, type d'activités sportives, maladies génétiques, historique de pathologies familiales, résultats de biochimie sanguine, résultats d'anciens spermogrammes, informations des difficultés d'érection, informations sur des pathologies testiculaires ou assimilées, nombre d'enfants, nombre d'accouchements prématurés, régularité du cycle menstruel, longueur du cycle menstruel, nombre de rapports sexuels hebdomadaires, historique de vaccination, historique sur des maladies sexuellement transmissibles.

L'ensemble de données contextuelles et/ou psycho-sociales fournies comporte des informations du patient, notamment des données du patient dont l'état psychologique, contexte culturel, niveau d'éducation, niveau de revenu et statut économique, environnement, profession, structure familiale.

L'étape 1 de fourniture d'un ensemble de données cliniques et de données contextuelles et/ou psycho-sociales comprend une sous étape (non représentée) de récolte de données, dites données autogénérées, relatives à l'utilisation faite par le patient du premier terminal informatique lors de l'exécution du questionnaire dynamique. Avantageusement, les données récoltées pourront notamment être des mesures du temps passé sur une question donnée, l'enregistrement de l'historique de navigation dans le questionnaire et, notamment, à partir du déplacement à travers les différentes questions du questionnaire, l'historique de navigation sur un moteur de recherche, horaire de connexion et de remplissage du questionnaire, nombre de connexions.

La fourniture de tout ou partie des données cliniques et de données contextuelles et/ou psycho-sociales du patient déclenche le calcul d'un score de littératie en santé, ledit score de littératie en santé étant associé au patient est stockée sur la mémoire de stockage 102 du serveur de traitement 101.

La valeur du score de littératie d'un patient est recalculée et mise à jour dans la mémoire chargée de sauvegarder sa valeur chaque fois que le patient interagit avec la première interface graphique exécutée dans le premier terminal informatique.

Le serveur de traitement 101 détermine le score de littératie en santé du patient de sorte que, en fonction dudit score et des données cliniques et des données contextuelles et/ou psycho-sociales fournies, le serveur de traitement 101 est apte à générer une instruction d'affichage d'une infobulle 202 sur le premier terminal informatique 103. Ladite infobulle 202 contient des informations sur les recommandations générées pour le patient en fonction des données cliniques et de données contextuelles et/ou psycho-sociales et, le cas échéant, des anciennes valeurs du score de littératie en santé.

Le procédé comporte une étape 2 d'identification, par le serveur de traitement 101, d'un ou plusieurs facteurs de risque d'infertilité à partir des données cliniques et de données contextuelles et/ou psycho-sociales fournies par le patient, un ensemble de règles d'identification de risques d'infertilité 105 étant stocké dans la mémoire de stockage 102, le ou lesdits facteurs de risque d'infertilité étant déterminés par le serveur de traitement 101 par application de tout ou partie des règles d'identification sur au moins une partie des données cliniques.

Le procédé comporte également une sous-étape de sélection 2.1 d'une ou plusieurs règles d'identification parmi l'ensemble de règles d'identification 105 à partir de tout ou partie de l'ensemble de données cliniques et de données contextuelles et/ou psycho-sociales fourni par le patient.

Le procédé comporte également une sous-étape 2.2 de filtrage de la bibliothèque de facteurs de risque d'infertilité prédéterminés 106 à partir des règles d'identification sélectionnées pour identifier le ou les facteurs de risque d'infertilité.

Le procédé comporte également une étape (non représentée), préalable à l'étape d'identification, de réception par le serveur de traitement 101 d'un deuxième ensemble de données cliniques et de données contextuelles et/ou psycho-sociales du patient correspondant à un dossier national de santé, le dit deuxième ensemble de données cliniques et de données contextuelles et/ou psycho-sociales étant transmis par un serveur de traitement distant (non représenté).

Le procédé comporte une étape 3 de génération par le serveur de traitement 101 d'un ensemble de recommandations de santé à partir des facteurs de risque d'infertilités identifiés et de l'ensemble de données cliniques et de données contextuelles et/ou psycho-sociales fourni.

Le procédé comporte une sous-étape 3.1 d'identification d'étiquettes à partir des facteurs de risque d'infertilité identifiés.

Le procédé comporte une sous-étape 3.2 de sélection par une ou plusieurs méthodes de maximisation d'un degré de correspondance sémantique d'une ou plusieurs recommandations de santé parmi une base de recommandations de santé 107 composée notamment d'articles scientifiques et de directives de santé publiques.

Le procédé comporte une étape 4 de transmission des données cliniques, du ou des facteurs de risque d'infertilité identifiés et des recommandations générées par le serveur de traitement 101 au deuxième terminal informatique 104.

Le procédé comporte une étape d'affichage 5, ultérieure à l'étape de transmission 4, d'un tableau de bord 109 contenant les données cliniques et de données contextuelles et/ou psycho-sociales et/ou du ou des facteurs de risque d'infertilité identifiés et/ou des recommandations générées par le serveur de traitement 101.

Le tableau de bord 109 comporte un premier composant graphique représentant temporellement l'ensemble des parcours de diagnostic effectués par le patient, les parcours effectués étant identifiés à partir de tout ou partie des données cliniques et de données contextuelles et/ou psycho-sociales fournies par le patient.

Le tableau de bord 109 comporte également un deuxième composant graphique représentant les recommandations générées par le serveur de traitement.

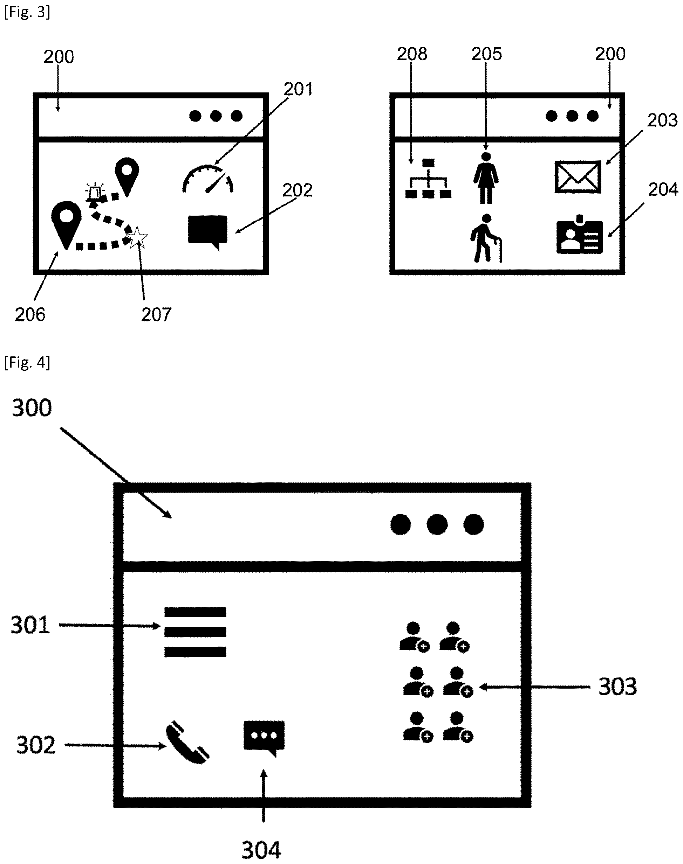

On a représenté en [Fig. 3] une interface graphique 200 du premier terminal informatique 103 selon deux modes de réalisation de l'invention.

Le premier terminal informatique est un ordinateur fixe comportant un écran.

Lors de l'étape de fourniture d'un ensemble de données cliniques, le patient procède au renseignement de l'ensemble de données cliniques et de données contextuelles et/ou psycho-sociales au moyen d'une première interface graphique affichée 200 dans le premier terminal informatique 103.

La première interface graphique est apte à afficher un ou plusieurs éléments graphiques, notamment des éléments graphiques comportant des synthèses d'informations de santé, visant à améliorer la compréhension du patient en ce qui concerne son parcours de fertilité et, notamment, à améliorer son score de littératie en santé.

L'ensemble de données cliniques et de données contextuelles et/ou psycho-sociales fournies comporte des informations du patient, notamment des informations parmi l'âge, le sexe, allergies connues, pathologies connues, antécédents médicaux, groupe sanguin, résultats d'examens tels que des bilans hormonaux et des tests de qualité spermatique, traitement en cours, handicaps, protocoles de procréation médicalement assistée déjà suivis, historique de traitement contraceptif, nom et/ou type des traitements contraceptifs utilisés, la durée d'utilisation desdits traitements contraceptifs, date de début de l'utilisation de traitements contraceptifs, date d'arrêt de l'utilisation de traitements contraceptifs, dates et nombre d'anciennes grossesses et le nombre de mois de conception associés auxdites anciennes grossesses, présence et type de complications lors d'une grossesse passée, nombre de partenaires sexuels, utilisation de drogues, consommation d'alcool, type d'activités sportives, maladies génétiques, historique de pathologies familiales, résultats de biochimie sanguine, résultats d'anciens spermogrammes, informations des difficultés d'érection, informations sur des pathologies testiculaires ou assimilées, nombre d'enfants, nombre d'accouchements prématurés, régularité du cycle menstruel, longueur du cycle menstruel, nombre de rapports sexuels hebdomadaires, historique de vaccination, historique sur des maladies sexuellement transmissibles.

La première interface graphique 200 comprend également des fonctionnalités pour la prise de rendez-vous 203, la gestion des médicaments et autres traitements thérapeutiques la gestion des résultats d'examens. Ladite première interface peut également comprendre des fonctionnalités d'alertes 207, notamment en cas d'un rendez-vous médical dont l'échéance est proche, de traitement arrivant à échéance ou des tests médicaux à réaliser.

La première interface graphique 200 prévoit la possibilité de téléverser et/ou télécharger des images, des fichiers audio et/ou des fichiers vidéo, notamment pour partager le contenu aux professionnels de santé.

La première interface graphique 200 comporte des outils de sécurisation informatique et de protection de la confidentialité des données fournies au moyen de ladite première interface graphique 200, lesdites données sont encodées au moyen d'un chiffrement homomorphique.

La première interface graphique 200 comporte une carte interactive 206, par exemple, sous forme de frise chronologique ou de chemin, permettant de visualiser les différentes étapes du parcours déjà suivies, telles que les dates de réalisation d'analyses biologiques, les dates de début et de fin de menstruation, les dates d'inséminations, les dates de début et fin de traitements thérapeutiques et les dates de consultations médicales.

La carte interactive 206 est établie à partir de l'ensemble d'informations cliniques fourni, des facteurs de risque déterminés et des recommandations générées de sorte que l'ensemble des événements majeurs du parcours du patient sont représentés par un ou plusieurs icônes sur ladite carte interactive.

La carte interactive 206 permet de générer un ensemble de parcours envisageables, de sorte que chaque parcours envisageable soit constitué d'un ensemble d'étapes, notamment des analyses de laboratoire et/ou des diagnostics annexes, envisageables au regard des protocoles de fertilité existants.

Les informations du patient pourront être représentées, notamment, au travers d'icônes, notamment des graphiques en forme de jauge 201 ou de barre de progression, et/ou des codes de couleurs permettant d'identifier rapidement les différents types d'événements et une ou plusieurs des caractéristiques desdits évènements.

On a représenté en [Fig. 4] une interface graphique 300 du deuxième terminal informatique 104 selon un mode de réalisation de l'invention.

Le deuxième terminal informatique 104 comporte une deuxième interface graphique 300, notamment à destination du médecin en charge du suivi du patient. Ladite deuxième interface graphique 300 permet notamment l'affichage d'un tableau de bord contenant les données cliniques et de données contextuelles et/ou psycho-sociales et/ou des risques de fertilité identifiés et/ou des recommandations de santé générées par le serveur de traitement 101.

La deuxième interface graphique 300 est agencée pour résumer graphiquement l'ensemble des données transmis par le serveur de traitement 101 sur un ou plusieurs composants graphiques (301, 302, 303, 304), dits résumés graphiques.

Le ou les résumés graphiques des données fournies pouvant comporter des graphes de valeurs temporelles, notamment une représentation temporelle de l'ensemble des parcours de diagnostics effectués par le patient.

La deuxième interface graphique comporte également un composant graphique (non représenté) synthétisant les facteurs de risque d'infertilité identifiés par le serveur de traitement et un troisième composant graphique (non représenté) synthétisant les recommandations générées par le serveur de traitement.

La description qui précède explique clairement comment l'invention permet d'atteindre les objectifs qu'elle s'est fixée, à savoir générer des recommandations d'un parcours de procréation médicalement assistée et d'augmenter, en proposant un procédé de génération de recommandations d'un parcours de procréation médicalement assistée mis en oeuvre par un système informatique.

En tout état de cause, l'invention ne saurait se limiter aux modes de réalisation spécifiquement décrits dans ce document, et s'étend en particulier à tous moyens équivalents et à toute combinaison techniquement opérante de ces moyens. On pourra en particulier envisager d'exécuter différentes étapes du procédé sur des serveurs de traitement distincts, notamment des serveurs de traitement distants les uns des autres.

## Revendications

1. Procédé de génération de recommandations pour un parcours de procréation médicalement assisté d'un patient, le procédé étant mis en oeuvre par un système informatique comportant : un serveur de traitement (101) comportant une mémoire de stockage (102), un premier terminal informatique (103) et un deuxième terminal informatique (104), les deux terminaux informatiques (103) et (104) étant aptes à communiquer avec ledit serveur de traitement (101) ; le procédé étant **caractérisé en ce qu'**il comporte les étapes suivantes :
a. étape (1) de fourniture d'un ensemble de données cliniques et de données contextuelles et/ou psycho-sociales du patient, au moins une partie desdites données cliniques et desdites données contextuelles et/ou psycho-sociales étant fournie au travers d'un questionnaire dynamique (108) exécuté par le patient au moyen dudit premier terminal informatique (103) ;
b. étape (2) d'identification, par ledit serveur de traitement (101), d'un ou plusieurs facteurs de risque d'infertilité à partir des données cliniques et de données contextuelles et/ou psycho-sociales fournies par le patient, un ensemble de règles d'identification de risques d'infertilité (105) étant stocké dans la mémoire de stockage (102), le ou lesdits facteurs de risque d'infertilité étant déterminés par le serveur de traitement (101) par application de tout ou partie desdites règles d'identification sur au moins une partie des données cliniques et de données contextuelles et/ou psycho-sociales ;
c. étape (3) de génération, par ledit serveur de traitement (101), d'un ensemble de recommandations de santé à partir des facteurs de risque d'infertilité identifiés et des données cliniques et de données contextuelles et/ou psycho-sociales fournies ;
d. étape (4) de transmission desdites données cliniques et desdites données contextuelles et/ou psycho-sociales, du ou des facteurs de risque d'infertilité identifiés et des recommandations générées par le serveur de traitement (101) audit deuxième terminal informatique (104).

2. Procédé selon la revendication précédente, **caractérisé en ce qu'**une bibliothèque de facteurs de risque d'infertilité prédéterminés (106) est stockée dans la mémoire de stockage (102) du serveur de traitement; **en ce que** chaque règle d'identification associe un ensemble de types de données cliniques et de données contextuelles et/ou psycho-sociales à un ou plusieurs facteurs de risque d'infertilité prédéterminés et à un parcours de diagnostic d'un patient ; et **en ce que** l'étape d'identification d'un ou plusieurs facteurs de risque d'infertilité comporte une sous-étape de sélection (2.1) d'une ou plusieurs règles d'identification parmi ledit ensemble de règles d'identification de facteurs de risque d'infertilité prédéterminés (106) à partir de tout ou partie de l'ensemble de données cliniques et de données contextuelles et/ou psycho-sociales fourni par le patient; et une sous-étape de filtrage (2.2) de ladite bibliothèque de facteurs de risque d'infertilité prédéterminés (106) à partir des règles d'identification sélectionnées pour identifier le ou lesdits facteurs de risque d'infertilité.

3. Procédé selon la revendication 2, **caractérisé en ce que** l'étape 3 de génération de recommandations de santé comprend une première sous-étape d'identification d'étiquettes (3.1) à partir desdits facteurs de risque d'infertilité identifiés et une deuxième sous-étape de sélection (3.2) par une ou plusieurs méthodes de maximisation d'un degré de correspondance sémantique d'une ou plusieurs recommandations de santé parmi une base de données de recommandations de santé (105).

4. Procédé selon la revendication 3, **caractérisé en ce que** la méthode de maximisation d'un degré de correspondance sémantique est une méthode de filtrage basée sur le contenu, une méthode de filtrage collaboratif, une méthode basée sur des graphes ou encore une méthode hybride composant plusieurs des méthodes précédentes; et **en ce que** la base de données de recommandations de santé (105) est composée d'articles scientifiques et/ou des directives de santé publiques.

5. Procédé selon l'une des revendications précédentes, **caractérisé en ce qu'**il comporte une étape d'affichage, ultérieure à l'étape de transmission au deuxième terminal informatique (104), d'un tableau de bord (109) contenant les données cliniques, et/ou, du ou des facteurs de risque d'infertilité identifiés et/ou des recommandations générées par le serveur de traitement (101).

6. Procédé selon la revendication 5, **caractérisé en ce que** chaque règle d'identification associe un ensemble de types de données cliniques et de données contextuelles et/ou psycho-sociales à un ou plusieurs facteurs de risque d'infertilité prédéterminés et à un parcours de diagnostic d'un patient, et **en ce que** le tableau de bord (109) affiché comporte un premier composant graphique représentant temporellement l'ensemble des parcours de diagnostic effectués par le patient, lesdits parcours effectués étant identifiés à partir de tout ou partie des données cliniques et de données contextuelles et/ou psycho-sociales fournies par le patient, un deuxième composant graphique représentant les facteurs de risque d'infertilité identifiés par le serveur de traitement (101) et un troisième composant graphique (109) représentant les recommandations générées par le serveur de traitement (101).

7. Procédé selon l'une des revendications précédentes, **caractérisé en ce qu'**il comporte une étape, préalable à l'étape d'identification, de réception par le serveur de traitement (101) d'un deuxième ensemble de données cliniques et de données contextuelles et/ou psycho-sociales du patient, notamment un profil national de santé ; ledit deuxième ensemble de données cliniques et de données contextuelles et/ou psycho-sociales étant transmis par un serveur de traitement distant.

8. Procédé selon l'une des revendications précédentes **caractérisé en ce que** l'étape de fourniture d'un ensemble de données cliniques et de données contextuelles et/ou psycho-sociales par le patient comprend une étape de récolte de données, dites autogénérées, relatives à l'utilisation du premier terminal informatique (103) faite par le patient lors de l'exécution du questionnaire dynamique (108), les données récoltées pourront notamment être des données parmi : mesure du temps d'utilisation de l'interface, mesure du temps passé sur une question, enregistrement de l'historique de navigation dans le questionnaire dynamique (108), notamment des déplacements à travers les différentes questions du questionnaire dynamique (108).

9. Procédé selon l'une des revendications précédentes, **caractérisé en ce qu'**il comporte une étape de calcul d'un score de littératie en santé à partir de l'ensemble de données cliniques et de données contextuelles et/ou psycho-sociales fourni et/ou des facteurs de risques identifiés et/ou des recommandations générées et/ou des donnés autogénérées ; et **en ce que** le serveur de traitement (101) est apte à envoyer un signal aux terminaux informatiques (103, 104) basé sur ledit score de littératie en santé.

10. Procédé selon la revendication 9, **caractérisé en ce qu'**il comporte une étape d'affichage, exécuté parallèlement à l'ensemble des étapes, sur le premier terminal informatique (103) d'un premier panneau, dit panneau de gestion, contenant au moins une partie des données cliniques et de données contextuelles et/ou psycho-sociales et/ou au moins une partie des facteurs de risque d'infertilité identifiés et/ou de recommandations générées par le serveur.
